(19) Europäisches Patentamt
European Patent Office
Office européen des brevets

(11) **EP 1 943 524 B1**

(12) **EUROPÄISCHE PATENTSCHRIFT**

(45) Veröffentlichungstag und Bekanntmachung des Hinweises auf die Patenterteilung:
**12.08.2009 Patentblatt 2009/33**

(51) Int Cl.:
***G01N 33/68*** *(2006.01)*

(21) Anmeldenummer: **06806361.9**

(22) Anmeldetag: **18.10.2006**

(86) Internationale Anmeldenummer:
**PCT/EP2006/010044**

(87) Internationale Veröffentlichungsnummer:
**WO 2007/045453 (26.04.2007 Gazette 2007/17)**

(54) **VERFAHREN ZUR CHARAKTERISIERUNG DER GLYCOSILIERUNG VON SIALOGLYCOPROTEINEN ÜBER EINE ISOFORMENZAHL I**

METHOD FOR CHARACTERIZING THE GLYCOSYLATION OF SIALOGLYCOPROTEINS VIA AN ISOFORM NUMBER I

PROCÉDÉ DE CARACTÉRISATION DE LA GLYCOSILATION DE SIALOGLYCOPROTÉINES SUR LA BASE DU NOMBRE D'ISOFORMES I

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.10.2005 DE 102005050580**

(43) Veröffentlichungstag der Anmeldung:
**16.07.2008 Patentblatt 2008/29**

(73) Patentinhaber: **Hermentin, Peter**
**35041 Marburg (DE)**

(72) Erfinder: **Hermentin, Peter**
**35041 Marburg (DE)**

(74) Vertreter: **Pfenning, Meinig & Partner GbR**
**Patent- und Rechtsanwälte**
**Theresienhöhe 13**
**80339 München (DE)**

(56) Entgegenhaltungen:
**WO-A-97/05490**

• **HERMENTIN P ET AL: "THE HYPOTHETICAL N-GLYCAN CHARGE: A NUMBER THAT CHARACTERIZES PROTEIN GLYCOSYLATION" GLYCOBIOLOGY, IRL PRESS,, GB, Bd. 6, Nr. 2, 1. März 1996 (1996-03-01), Seiten 217-230, XP000610494 ISSN: 0959-6658**

• **HERMENTIN P ET AL: "THE HYPOTHETICAL N-GLYCAN CHARGE. A NUMBER TO CHARACTERIZE PROTEIN N-GLYCOSYLATION" PHARMACY AND PHARMACOLOGY COMMUNICATIONS, LONDON, GB, Bd. 5, Nr. 1, Januar 1999 (1999-01), Seiten 33-43, XP009078630 ISSN: 1460-8081**

• **HERMENTIN PETER ET AL: "A strategy for the mapping of N-glycans by high-performance capillary electrophoresis" ANALYTICAL BIOCHEMISTRY, Bd. 221, Nr. 1, 1994, Seiten 29-41, XP002419846 ISSN: 0003-2697**

• **GOLDMAN M H ET AL: "Monitoring recombinant human interferon-gamma N-glycosylation during perfused fluidized-bed and stirred-tank batch culture of CHO cells" BIOTECHNOLOGY AND BIOENGINEERING, WILEY & SONS, HOBOKEN, NJ, US, Bd. 60, Nr. 5, 5. Februar 1998 (1998-02-05), Seiten 596-607, XP002353272 ISSN: 0006-3592**

• **KELLY J F ET AL: "Development of electrophoretic conditions for the characterization of protein glycoforms by capillary electrophoresis-electrospray mass spectrometry" JOURNAL OF CHROMATOGRAPHY A, ELSEVIER, AMSTERDAM, NL, Bd. 720, Nr. 1, 12. Januar 1996 (1996-01-12), Seiten 409-427, XP004038372 ISSN: 0021-9673**

EP 1 943 524 B1

**Beschreibung**

Gegenstand der vorliegenden Erfindung ist ein Verfahren zur Charakterisierung der Qualität von Sialoglycoproteinen sowie ein in-vitro Verfahren zur Bestimmung der Bioverfügbarkeit von Sialoglycoproteinen mittels der Kapillarzonenelektrophorese (CZE.), das auf der "Isoformenzahl", im folgenden I genannt, basiert und sowohl für endogene Glycoproteine als auch exogene Glycoproteine eingesetzt werden kann.

Exogene Glycoproteine sind in diesem Zusammenhang z.B. rekombinante, aus Säugerzellen gewonnene therapeutische Glycoproteine, wie z. B. Erythropoietin, Follikel-stimulierendes Hormon, Chorion-Gonadotropin, Gewebsplasminogen-Aktivator oder Antithrombin III.

Endogene Glycoproteine sind in diesem Zusammenhang humane oder nicht humane, z. B. bovine, Serumglycoproteine, wie z. B. humanes $\alpha_1$-saures Glycoprotein, humanes Transferrrin oder bovines Fetuin.

Die Erforschung, Entwicklung und Produktion therapeutischer Glycoproteine sowie ihre behördliche bzw. klinische Zulassung erfordert eine aufwändige Analytik im Hinblick auf die in vivo-Halbwertszeit, biologische Sicherheit, Produkt-Definition und Chargenkonsistenz.

In dieser Hinsicht spielt bislang vor allem der Anteil an Sialinsäure als Parameter eine wichtige Rolle, da bekannt ist, dass das Vorhandensein bzw. Fehlen von Sialinsäure die Zirkulations-Halbwertszeit eines Glycoproteins im Blut bzw. seine Clearance entscheidend mitbestimmt.

Bei Erythropoietin (EPO) stellt die Kapillarzonenelektrophorese (CZE) eine wichtige Methode dar, das Sialoglycoprotein zu charakterisieren und seine biologische Wirksamkeit zu belegen. Diese Prüfung ist in der Europäischen Pharmakopoe (Ph. Eur.) beschrieben und für jede klinische Charge von EPO als Freigabe-relevanter Test gefordert.

Derzeit ist man bestrebt, die biologische Referenzpräparation 1 (BRP1) von EPO durch die biologische Referenzpräparation 2 (BRP2) zu ersetzen. Hierzu wurde BRP1 von EPO im Rahmen einer Ringstudie mit der "candidate biological reference preparation 2" (cBRP2) von EPO unter anderem mit Hilfe der Kapillarzonenelektrophorese vermessen und die dabei gewonnenen analytischen Daten detailliert verglichen (M-E. Behr-Gross, A. Daas, A.F. Bristow, Collaborative study for the establishment of erythropoietin BRP batch 2, Pharmeuropa Bio 2004-1, 23-33).

Der vorliegenden Erfindung lag somit die Aufgabe zugrunde, ein Verfahren bereitzustellen, welches erlaubt, die komplexen Daten der Kapillarzonenelektrophorese in einer im Vergleich zur Europäischen Pharmakopoe bzw. zu Behr-Gross et al. (2004) vergleichsweise vereinfachten und dennoch aussagekräftigen, verlässlichen und validen Weise darzustellen, wobei das Verfahren geeignet sein sollte, die bekannten in-vivo Verfahren z.B. zur Bestimmung der Bioverfügbarkeit und der Chargenkonsistenz von Sialoglycoproteinen zu vereinfachen bzw. zu ersetzen.

Diese Aufgabe wird durch das Verfahren mit den Merkmalen des Anspruchs 1 gelöst. Die weiteren abhängigen Ansprüche zeigen vorteilhafte Weiterbildungen auf. Im Anspruch 18 werden erfindungsgemäße Verwendungen genannt.

Es wurde überraschender Weise festgestellt, dass die über die CZE eines Sialoglycoproteins ermittelte Isoformenzahl I hervorragend mit der in-vivo Bioverfügbarkeit und biologischen Aktivität eines Sialoglycoproteins korreliert. Wegen der guten Reproduzierbarkeit und der analytischen Genauigkeit dieser Bestimmung kann I vorteilhafter Weise auch in einem Verfahren zum Beleg der Chargenkonsistenz eines therapeutischen Glycoproteins eingesetzt werden.

Die vorliegenden Untersuchungen lassen den Schluss zu, dass sich der in der CZE sichtbar gemachte "Isoformen-Status" und somit der "Glycosylierungs-Status" eines Sialoglycoproteins in der einfachen Zahl I sehr hilfreich und aussagekräftig beschreiben lässt. Daher kann mittels der Bestimmung von I die Isoformenverteilung eines Glycoproteins von Charge zu Charge oder im Rahmen von Stabilitätsstudien auf einfachem Wege verglichen werden.

Unter dem "Glycosylierungs-Status" eines Glycoproteins versteht man im Zusammenhang mit der vorliegenden Erfindung die Zusammensetzung der Glycane des Glycoproteins aus bi-, tri- und tetraantennären Oligosaccharid-Strukturen und ihrem jeweiligen Sialylierungsgrad, d.i. der Gehalt an gebundener Sialinsäure, sowie dem Gehalt an Sulfat-oder PhosphatGruppen, einschließlich des Glycosylierungsbeitrags der O-glycosidisch verknüpften sialylierten Zuckerketten.

Unter der Bioverfügbarkeit eines Glycoprotein-Therapeutikums versteht man die Fähigkeit des Therapeutikums, seine biologische Aktivität bzw. therapeutische Wirksamkeit in vivo zu entfalten. Demnach werden die Bioverfügbarkeit und die biologische Aktivität maßgeblich vom in vivo-Clearance-Verhalten, also der Entfernung des Therapeutikums aus der Blutzirkulation bestimmt. Beispielsweise ist für EPO bekannt, dass es bei einem Fehlen der in den N-glycosidischen Zuckerketten endständig gebundenen N-Acetylneuraminsäure sehr schnell über den sogenannten "asialo-Rezeptor" in der Leber aus der Blutzirkulation entfernt wird und somit seine biologische Wirksamkeit nicht entfalten kann.

Die Isoformenzahl I eines therapeutischen Glycoproteins korreliert in überraschender Weise mit der in vivo-Halbwertszeit des Glycoproteins und stellt somit einen völlig neuen Messparameter dar, der es erlaubt, das für das therapeutische Glycoprotein von Charge zu Charge zu erwartende Clearance-Verhalten auf sehr einfache Art und Weise im Voraus abzuschätzen. Infolgedessen ermöglicht I auch eine Aussage über die für das Glycoprotein von Charge zu Charge zu erwartende biologische Wirksamkeit. Daher kann man bei Ermittlung von I eines therapeutischen Glycoproteins von Charge zu Charge z. B. auf die sehr aufwändige, zeitraubende, teure und recht ungenaue Ermittlung der therapeutischen Wirksamkeit des Glycoproteins in Tierexperimenten (in-vivo Assay) verzichten.

Dies ermöglicht zudem einen neuen und erheblichen Beitrag zur Reduktion von Tierexperimenten und somit zu einem verbesserten Tierschutz. Gleichzeitig stellt die Isoformenzahl I ein besonders geeignetes Maß für die Chargenkonsistenz des Glycoproteins dar.

Die Berechnung der Isoformenzahl I eines Sialoglycoproteins erfolgt dadurch, dass zunächst das Sialoglycoprotein mittels Kapillarzonenelektrophorese aufgetrennt wird. Aufbauend auf der Auswertung der Kapillarzonenelektrophorese wird dann jeweils ein Produkt $i_n = m_n \cdot p_n$ aus dem über die Kapillarzonenelektrophorese erhaltenen prozentualen Peakflächenanteil p der Isoform n ($p_n$) mit einem Multiplikator $m_n$ gebildet, wobei n eine ganze Zahl von 1 bis 14, $m_n = x_n \cdot n$ und $x_n$ eine beliebige Zahl aus der Gruppe der reellen Zahlen außer 0 ist. Die so erhaltenen Produkte $i_1$ bis $i_n$ werden anschließend zur Isoformenzahl I

$$I = \sum_{i=1}^{i=n} i_n$$

aufsummiert. Die Summe der prozentualen Isoformenanteile ergibt 100%.

Beispielsweise ist $x_n$ für jede Isoform = 1, wodurch der Multiplikator der Zahl der entsprechenden Isoform entspricht.

Beispielsweise kann $x_n$ jedoch für jede Isoform auch verschieden und ungleich 1 sein, wodurch der Multiplikator für jede Isoform verschieden ist. Vorzugsweise ist $x_n$ dabei eine variable Zahl, welche sich aus der spezifischen biologischen Aktivität der jeweiligen Isoform errechnet. Die spezifische Aktivität der Isoformen z.B. von rekombinantem Erythropoietin aus CHO-Zellen findet sich z.B. in EP 0 428 267 B1 (1997; Priorität US 421444 (1989).

Die Bestimmung von I wurde an verschiedenen Chargen eines therapeutischen Glycoproteins verifiziert und auf den literaturbekannten Vergleich von EPO-BRP1 mit EPO-cBRP2 (M-E. Behr-Gross, A. Daas, A.F. Bristow, Collaborative study for the establishment of erythropoietin BRP batch 2, Pharmeuropa Bio 2004-1, 23-33) übertragen. Dabei konnte gezeigt werden, dass die Isoformenzahl I als ein neuer, aussagekräftiger, verlässlicher und charakteristischer Parameter für die Isoformen-Verteilung bzw. die Protein-Glycosylierung angesehen werden kann.

Anhand der nachfolgenden Beispiele soll das erfindungsgemäße Verfahren näher beschrieben werden, ohne dieses auf die hier dargestellten Ausführungsformen beschränken zu wollen.

Im Folgenden findet sich als Beispiel die Bestimmung von I aus der Collaborative Study von EPO (Behr-Gross et al., 2004). Hierzu wurden die in Behr-Gross et al. (2004) publizierten Daten ausgewertet. Die in der Studie erhaltenen Ergebnisse sind für BRP1 in Tabelle 1 bzw. Tabelle 3 und für cBRP2 in Tabelle 2 bzw. Tabelle 4 zusammengefasst.

Die Studie hat leichte Unterschiede zwischen BRP1 und cBRP2 aufgezeigt, welche nach Auffassung von Behr-Gross et al. (2004) eine Anpassung der Ph. Eur.-Monografie erforderlich machen:

Der Anteil an Isoform 3 ist in cBRP2 im Schnitt geringer als von der Ph.Eur. -Monografie gefordert, während Isoform 7 sehr knapp unterhalb der Obergrenze der aktuellen Ph.Eur.-Spezifikation liegt.

Die vorliegende Erfindung zur in-vitro-Bewertung der Qualität und biologischen Aktivität von EPO im Besonderen bzw. der Qualität von Sialoglycoproteinen im Allgemeinen hat gegenüber dem Stand der Technik (Hermentin et al. (1996) Glycobiology 6, 217-230; EP 0843821 B1 (2001), Priorität DE 19527054 vom 26.07.1995) den ausgesprochenen Vorteil, dass sich die Isoformenbestimmung bereits mit dem nativen Glycoprotein und in einem normalen Proteinlabor durchführen lässt, sofern das Labor über eine Kapillarelektrophorese-Apparatur verfügt. Hingegen erfordert der Stand der Technik zur Bestimmung der hypothetischen Ladungsxahl Z eines Glycoproteins, mit deren Hilfe die biologische Aktivität von EPO gleichfalls, jedoch in weit schwierigerer Weise ermittelt werden kann (Hermentin et al., ibid.) eine ausgesprochene Expertise zur Freisetzung und Isolierung des N-Glycan-Pools (der N-glycosidisch gebundenen Zuckerketten) des Glycoproteins, welcher dann mittels Anionenaustauschchromatographie, insbesondere mittels "high-pH anion-exchange chromatography with pulsed amperometric detection" (HPAEC-PAD) ladungsmäßig aufgetrennt und analysiert wird (Hermentin et al., ibid.).

Die Isoformenzahl I, deren Ermittlung bereits mit dem intakten Glycoprotein erfolgt, vermag eine ähnlich aussagekräftige Information über die biologische Wirksamkeit, biologische Halbwertszeit, Stabilität und Chargenkonsistenz von EPO im Allgemeinen bzw. von Sialoglycoproteinen, welche N-Glycane tragen, im Besonderen zu vermitteln, wie die hypothetische Ladungszahl Z (Hermentin et al., ibid.), deren Bestimmung jedoch eine deutlich aufwändigere und anspruchsvoller Technik und Expertise erfordert.

Solange die Kapillarelektrophorese von EPO von der Europäischen Pharmakopöe (Monograph 1316; Ph. Eur. Suppl. 5.3. (Erythropoietin concentrated solution), Strasbourg, France, Council of Europe; 2005) als Freigabetest für Erythropoietin verbindlich vorgeschrieben ist, so lange wird die hier erfindungsgemäß vorgestellt Isoformenzahl I einen einfachen, schlüssigen und verlässlichen Bewertungs- und Freigabeparameter für die Qualität von EPO-Präparaten, das heißt, deren biologische Wirksamkeit, biologische Halbwertszeit, Chargenkonsistenz und Lagerstabilität darstellen können.

Die Erfindung wird durch die nachfolgenden Beispiele 1 bis 4 näher erläutert.

**Beispiel 1**

Berechnung der Isoformenzahl I für EPO-BRP1 anhand der von Behr-Gross et al. (2004) publizierten Daten der Isoformenverteilung von EPQ-BRP1; dabei gilt für das Produkt $i_n=m_n \cdot p_n$ mit $m_n=x_n \cdot n$ für jede Isoform $x_n=1$; n entspricht der jeweiligen Isoformen-Nummer.

Tabelle 1

| BRP1 | Lab 2 | | Lab 3 | | Lab 8 | | Lab 9 | | Lab 10 | | Lab 11 | |
|---|---|---|---|---|---|---|---|---|---|---|---|---|
| $I\,0$ | Area % | $i_n$ | Area % | $i_n$ | Area % | $i_n$ | Area % | $i_n$ | Area % | $i_n$ | Area % | $i_n$ |
| $I\,1$ | 0, 4 | 0,4 | 0,8 | 0,8 | 1,0 | 1,0 | 0,8 | 0,8 | 0,8 | 0,8 | 1,0 | 1,0 |
| $I\,2$ | 1,9 | 3,8 | 2,5 | 5,0 | 3,1 | 6,2 | 2,2 | 4,4 | 2,4 | 4,8 | 2,7 | 5,4 |
| $I\,3$ | 5,9 | 17,7 | 6,0 | 18,0 | 6,9 | 20,7 | 5,6 | 16,8 | 6,2 | 18,6 | 6,2 | 18,6 |
| $I\,4$ | 18,4 | 73,6 | 18,3 | 73,2 | 17,8 | 71,2 | 19,1 | 76,4 | 17,7 | 70,8 | 19,0 | 76,0 |
| $I\,5$ | 29,6 | 148,0 | 29,0 | 145,0 | 28,2 | 141,0 | 30,1 | 150,5 | 28,8 | 144,0 | 28,8 | 144,0 |
| $I\,6$ | 28,7 | 172,2 | 27,9 | 167,4 | 27,5 | 165,0 | 24,4 | 146,4 | 28,5 | 171,0 | 27,8 | 166,8 |
| $I\,7$ | 14,4 | 100,8 | 14,2 | 99,4 | 14,1 | 98,7 | 16,1 | 112,7 | 14,6 | 102,2 | 13,7 | 95,9 |
| $I\,8$ | 0,7 | 5,6 | 1,3 | 10,4 | 1,4 | 11,2 | 1,8 | 14,4 | 0,9 | 7,2 | 0,8 | 6,4 |
| total | 100,0 | **522,1** | 100,0 | **519,2** | 100,0 | **515,0** | 100,1 | **522,4** | 99,9 | **519,4** | 100,0 | **514,1** |

Wie aus Tabelle 1 ersichtlich, konnten für BRP1 aus den Daten der sechs Labore, die an der Collaborative Study teilnahmen, folgende Isoformenzahlen ermittelt werden:

| | | |
|---|---|---|
| Lab 2: | I = 522,1 | (gerundet: I = 522) |
| Lab 3: | I = 519,2 | (gerundet: I = 519) |
| Lab 8: | I = 515,0 | (gerundet: I = 515) |
| Lab 9: | I = 522,4 | (gerundet: I = 522) |
| Lab 10: | I = 519,4 | (gerundet: I = 519) |
| Lab 11: | I = 514,1 | (gerundet: I = 514) |

| | | |
|---|---|---|
| Mittel: | I = 518,7 | (gerundet: I = 519) |
| Standardabweichung: | Stabw = 3,5 | |
| Variationskoeffizient: | VK = 0,7 % | |

Die Daten von Tabelle 1 zeigen, dass sich, wenn für jede Isoform $x_n=1$ ist, aus den CZE-Daten für BRP1 der sechs Labore, die an dieser Collaborative Study teilgenommen haben, eine Isoformenzahl I zwischen 514 und 522 errechnen lässt, was die gute Vergleichbarkeit der CZE-Analyse von Labor zu Labor sowie die hohe Präzision der Isoformenzahl I belegt.

**Beispiel 2**

Berechnung der Isoformenzahl I für EPO-cBRP2 anhand der von Behr-Gross et al. (2004) publizierten Daten der Isoformenverteilung von EPO-cBRP2; dabei gilt für das Produkt $i_n=m_n \cdot p_n$ mit $m_n=x_n \cdot n$ für jede Isoform $x_n=1$; n entspricht der jeweiligen Isoformen-Nummer.

**Tabelle 2**

| cBRP2 | Lab 2 | | Lab 3 | | Lab 8 | | Lab 9 | | Lab 10 | | Lab 11 | | Lab 12 | |
|---|---|---|---|---|---|---|---|---|---|---|---|---|---|---|
| I 0 | Area % | $i_n$ | Area % | $i_n$ | Area % | $i_n$ | Area % | $i_n$ | Area % | $i_n$ | Area % | $i_n$ | Area % | $i_n$ |
| I 1 | | | | | 0,3 | 0,3 | 0,5 | 0,5 | 0,4 | 0,4 | 0,3 | 0,3 | 0,2 | 0,2 |
| I 2 | 1,0 | 2,0 | 1,0 | 2,0 | 1,6 | 3,2 | 0,9 | 1,8 | 1,3 | 2,6 | 1,5 | 3,0 | 1,0 | 2,0 |
| I 3 | 4,5 | 13,5 | 4,9 | 14,7 | 5,1 | 15,3 | 3,8 | 11,4 | 4,6 | 13,8 | 5,2 | 15,6 | 5,3 | 15,9 |
| I 4, | 16,8 | 67,2 | 16,8 | 67,2 | 16,2 | 64,8 | 17,3 | 69,2 | 15,4 | 61,6 | 19,0 | 76,0 | 17,3 | 69,2 |
| I 5 | 27,1 | 135,5 | 27,2 | 136,0 | 26,3 | 131,5 | 27,8 | 139,0 | 26,8 | 134,0 | 28,7 | 143,5 | 26,9 | 134,5 |
| I 6 | 30,4 | 182,4 | 29,7 | 178,2 | 30,0 | 180,0 | 29,4 | 176,4 | 30,4 | 182,4 | 30,0 | 180,0 | 29,6 | 177,6 |
| I 7 | 18,6 | 130,2 | 18,3 | 128,1 | 18,6 | 130,2 | 18,6 | 130,2 | 19,2 | 134,4 | 15,2 | 106,4 | 18,1 | 126,7 |
| I 8 | 1,7 | 13,6 | 2,1 | 16,8 | 1,8 | 14,4 | 1,8 | 14,4 | 1,9 | 15,2 | 0,1 | 0,8 | 1,7 | 13,6 |
| **Total** | 100,1 | **544,4** | 100,0 | **543,0** | 99,9 | **539,7** | 100,1 | **542,9** | 100,0 | **544,4** | 100,0 | **525,6** | 100,1 | **539,7** |

Wie aus Tabelle 2 ersichtlich, konnten für cBRP2 aus den Daten der sieben Labore, die an der Collaborative Study teilnahmen, folgende Isoformenzahlen ermittelt werden:

Lab 2:      I = 544,4     (gerundet: I = 544)
Lab 3:      I = 543,0     (gerundet: I = 543)
Lab 8:      I = 539,7     (gerundet: I = 540)
Lab 9:      I = 542,9     (gerundet: I = 543)
Lab 10:     I = 544,4     (gerundet: I = 544)
Lab 11:     I = 525,6     (gerundet: I = 526)
Lab 12:     I = 539,7     (gerundet: I = 540)

Mittelwert:                        MW = 540,0     (gerundet: I = 540)
Standardabweichung:                Stabw = 6,6
Varia.tionskoeffizient:            VK = 1,2 %

Aus den Daten von Tabelle 2 fällt auf, dass, wenn für jede Isoform $x_n=1$ ist, sich aus den CZE-Daten für cBRP2 der sieben Labore, die an dieser Collaborative Study teilgenommen haben - mit Ausnahme von Labor 11 -, eine Isoformenzahl I zwischen 540 und 544 errechnen lässt, was die gute Vergleichbarkeit der CZE-Analyse von Labor zu Labor sowie die hohe Präzision der Isoformenzahl I belegt.

Für Labor 11 fällt bei dieser gemäß der vorliegenden Erfindung möglichen Auswertung auf, dass die errechnete Isoformenzahl mit I = 526 signifikant niedriger liegt als in den übrigen Laboren (I = 540-544), was für Labor 11 für cBRP2 (im Vergleich zu den übrigen Laboren) eine leichte Abweichung (evtl. einen leichten Analysenfehler) nahelegt.

Dieser Umstand wird nur über diese gemäß der vorliegenden Erfindung mögliche Auswertung der Isoformenzahl I ersichtlich, was die hohe Aussagkraft dieser Isoformenzahl I hinsichtlich der Isoformenverteilung von EPO im Besonderen bzw. von Sialoglycoproteinen im Allgemeinen belegt.

Im Übrigen wird der in der Collaborative Study beschriebene Unterschied zwischen EPO-BRP1 und EPO-cBRP2 über die Isoformenzahl in einfacher und schlüssiger Form ersichtlich. Für EPO-BRP1 wurde eine mittlere Isoformenzahl von I = 519 ermittelt, während für EPO-cBRP2 eine mittlere Isoformenzahl von I = 540 errechnet wurde. Dieser Umstand belegt die hohe Aussagekraft der Isoformenzahl I hinsichtlich der Qualität von EPO im Allgemeinen bzw. von Sialoglycoproteinen im Besonderen und die Eignung der Isoformenzahl I als ein Qualitätsmerkmal für die Chargenkonsistenz, die Lagerstabilität, die biologische Halbwertszeit und die biologische Wirksamkeit eines therapeutischen Glycoproteins.

**Beispiel 3**

Berechnung der Isoformenzahl I für EPO-BRP1 anhand der von Behr-Gross et al. (2004) publizierten Daten der Isoformenverteilung von EPO-BRP1 unter Berücksichtigung der spezifischen Aktivität der einzelnen Isoformen gemäß EP 0 428 267 B1 (1996; Priorität US 421444 (1989)), basierend auf der Konzentrationsbestimmung bei 280 nm. Dabei gilt in dem Produkt $in = m_n \cdot p_n$ mit $m_n = x_n \cdot n$ für $x_n$ jeweils die in der Spalte U/mg genannte Zahl (die spezifische Isoformen-Aktivität [U/mg] gemäß EP 0 428 267 B1), dividiert durch 100; dabei gilt für n jeweils n = 1. Die jeweilige Isoformenzahl I reflektiert somit in besonders zutreffender Weise die biologische Aktivität des EPO-Produkts.

**Tabelle 3**

| BRP1 | | | Lab 2 | | Lab 3 | | Lab 8 | | Lab 9 | | Lab 10 | | Lab 11 | |
|---|---|---|---|---|---|---|---|---|---|---|---|---|---|---|
| I 0 | U/mg | $x_n$ | Area% | $i_n$ | Area% | $i_n$ | Area% | $i_n$ | Area% | $i_n$ | Area% | $i_n$ | Area% | $i_n$ |
| I 1 | 50300 | 503 | 0,4 | 201 | 0,8 | 402 | 1,0 | 503 | 0,8 | 402 | 0,8 | 402 | 1,0 | 503 |
| I 2 | 70600 | 706 | 1,9 | 1341 | 2,5 | 1765 | 3,1 | 2189 | 2,2 | 1553 | 2,4 | 1694 | 2,7 | 1906 |
| I 3 | 96600 | 966 | 5,9 | 5699 | 6,0 | 5796 | 6,9 | 6665 | 5,6 | 5410 | 6,2 | 5989 | 6,2 | 5989 |
| I 4 | 170300 | 1703 | 18,4 | 31335 | 18,3 | 31165 | 17,8 | 30313 | 19,1 | 32527 | 17,7 | 30143 | 19,0 | 32357 |
| I 5 | 255800 | 2558 | 29,6 | 75717 | 29,0 | 74182 | 28,2 | 72136 | 30,1 | 76996 | 28,8 | 73670 | 28,8 | 73670 |
| I 6 | 258400 | 2584 | 28,7 | 74161 | 27,9 | 72094 | 27,5 | 71060 | 24,4 | 63050 | 28,5 | 73644 | 27,8 | 71835 |
| I 7 | 258700 | 2587 | 14,4 | 37253 | 14,2 | 36735 | 14,1 | 36477 | 16,1 | 41651 | 14,6 | 37770 | 13,7 | 35442 |
| I 8 | 205800 | 2058 | 0,7 | 1441 | 1,3 | 2675 | 1,4 | 2881 | 1,8 | 3704 | 0,9 | 1852 | 0,8 | 1646 |
| total | | | 100,0 | **227148** | 100,0 | **224815** | 100,0 | **222224** | 100,1 | **225293** | 99,9 | **225166** | 100,0 | **223349** |

Wie aus Tabelle 3 ersichtlich, konnten für BRP1 aus den Daten der sechs Labore, die an der Collaborative Study teilnahmen, folgende gemäß der vorliegenden Erfindung ebenfalls möglichen alternativen Isoformenzahlen ermittelt werden:

|        |           |
|--------|-----------|
| Lab2:  | I = 227148 |
| Lab3:  | I = 224815 |
| Lab8:  | I = 222224 |
| Lab9:  | I = 225293 |
| Lab10: | I = 225166 |
| Lab11: | I = 223349 |

|                        |              |
|------------------------|--------------|
| Mittel:                | I = 224666   |
| Standardabweichung:    | Stabw = 1704 |
| Variationskoeffizient: | VK = 0,8 %   |

Die Daten von Tabelle 3 zeigen, dass sich aus den CZE-Daten für BRP1 der sechs Labore, die an dieser Collaborative Study teilgenommen haben, eine alternative (wahlweise mögliche) Isoformenzahl I zwischen 222224 und 227148 errechnen lässt, was die gute Vergleichbarkeit der CZE-Analyse von Labor zu Labor sowie die hohe Präzision der alternativ möglichen Isoformenzahl I ebenfalls belegt.

**Beispiel 4**

Berechnung der Isoformenzahl I für EPO-cBRP2 anhand der von Behr-Gross et al. (2004) publizierten Daten der Isoformenverteilung von EPO-cBRP2 unter Berücksichtigung der spezifischen Aktivität der einzelnen Isoformen gemäß EP 0 428 267 B1 (1996; Priorität US 421444 (1989), basierend auf der Konzentrationsbestimmung bei 280 nm. Dabei gilt in dem Produkt $i_n = m_n \cdot p_n$ mit $m_n = x_n \cdot n$ für $x_n$ jeweils die in der Spalte U/mg genannte Zahl (die spezifische Isoformen-Aktivität [U/mg] gemäß EP 0 428 267 B1), dividiert durch 100; dabei gilt.für n jeweils n = 1. Die jeweilige Isoformenzahl I reflektiert somit in besonders zutreffender Weise die biologische Aktivität des EPO-Produkts.

**Tabelle 4**

| cBRP2 | | | Lab 2 | | Lab 3 | | Lab 8 | | Lab 9 | | Lab 10 | | Lab 11 | | Lab 12 | |
|---|---|---|---|---|---|---|---|---|---|---|---|---|---|---|---|---|
| I 0 | U/mg | $x_n$ | Area% | $i_n$ | Area% | $i_n$ | Area% | $i_n$ | Area% | $i_n$ | Area% | $i_n$ | Area% | $i_n$ | Area% | $i_n$ |
| I 1 | 50300 | 503 | | 0 | | 0 | 0,3 | 151 | 0,5 | 252 | 0,4 | 201 | 0,3 | 151 | 0,2 | 101 |
| I 2 | 70600 | 706 | 1,0 | 706 | 1,0 | 706 | 1,6 | 1130 | 0,9 | 635 | 1,3 | 918 | 1,5 | 1059 | 1,0 | 706 |
| I 3 | 96600 | 966 | 4,5 | 4347 | 4,9 | 4733 | 5,1 | 4927 | 3,8 | 3671 | 4,6 | 4444 | 5,2 | 5023 | 5,3 | 5120 |
| I 4 | 170300 | 1703 | 16,8 | 28610 | 16,8 | 28610 | 16,2 | 27589 | 17,3 | 29462 | 15,4 | 26226 | 19,0 | 32357 | 17,3 | 29462 |
| I 5 | 255800 | 2558 | 27,1 | 69322 | 27,2 | 69578 | 26,3 | 67275 | 27,8 | 71112 | 26,8 | 68554 | 28,7 | 73415 | 26,9 | 68810 |
| I 6 | 258400 | 2584 | 30,4 | 78554 | 29,7 | 76745 | 30,0 | 77520 | 29,4 | 75970 | 30,4 | 78554 | 30,0 | 77520 | 29,6 | 76486 |
| I 7 | 258700 | 2587 | 18,6 | 48118 | 18,3 | 47342 | 18,6 | 48118 | 18,6 | 48118 | 19,2 | 49670 | 15,2 | 39322 | 18,1 | 46825 |
| I 8 | 205800 | 2058 | 1,7 | 3499 | 2,1 | 4322 | 1,8 | 3704 | 1,8 | 3704 | 1,9 | 3910 | 0,1 | 206 | 1,7 | 3499 |
| total | | | 100,1 | **233156** | 100,0 | **232036** | 99,9 | **230414** | 100,1 | **232924** | 100,0 | **232477** | 100,0 | **229053** | 100,1 | **231008** |

Wie aus Tabelle 4 ersichtlich, konnten für cBRP2 aus den Daten der sieben Labore, die an der Collaborative Study teilnahmen, folgende gemäß der vorliegenden Erfindung ebenfalls möglichen alternativen Isoformenzahlen ermittelt werden:

|        |             |
|--------|-------------|
| Lab2:  | I = 233156  |
| Lab3:  | I = 232036  |
| Lab8:  | I = 230414  |
| Lab9:  | I = 232924  |
| Lab10: | I = 232477  |
| Lab11: | I = 229053  |
| Lab12: | I = 231008  |

| Mittelwert:          | MW = 231581  |
|----------------------|--------------|
| Standardabweichung:  | Stabw = 1493 |
| Variationskoeffizient: | VK = 0,6 % |

Aus den Daten von Tabelle 4 fällt auf, dass sich aus den CZE-Daten für cBRP2 der sieben Labore, die an dieser Collaborative Study teilgenommen haben eine alternative (wahlweise mögliche) Isoformenzahl I zwischen 229053 und 233156 errechnen lässt, was die gute Vergleichbarkeit der CZE-Analyse von Labor zu Labor sowie die hohe Präzision der Isoformenzahl I ebenfalls belegt.

Gemäß dieser (wahlweise möglichen) Auswertung sind die Ergebnisse aus Labor 11 im Vergleich zu den übrigen Laboren unauffällig.

Im Übrigen wird der in der Collaborative Study beschriebene Unterschied zwischen EPO-BRP1 und EPO-cBRP2 auch über diese gemäß der vorliegenden Erfindung wahlweise mögliche Errechnung der Isoformenzahl in einfacher und schlüssiger Form ersichtlich. Für EPO-BRP1 wurde eine mittlere Isoformenzahl von I = 224666 ermittelt, während für EPO-cBRP2 eine mittlere Isoformenzahl von I = 231581 errechnet wurde. Auch diese gemäß der vorliegenden Erfindung wahlweise mögliche Errechnung der Isoformenzahl belegt die hohe Aussagekraft der Isoformenzahl hinsichtlich der Qualität von EPO im Allgemeinen bzw. von Sialoglycoproteinen im Besonderen und die Eignung der Isoformenzahl I als ein Qualitätsmerkmal für die Chargenkonsistenz, die Lagerstabilität, die biologische Halbwertszeit und die biologische Wirksamkeit eines therapeutischen Glycoproteins.

**Patentansprüche**

1. Verfahren zur Charakterisierung der Qualität von Sialoglycoproteinen durch eine Isoformenzahl I, bei dem

 a) das Sialoglycoprotein mittels Kapillarzonenelektrophorese aufgetrennt wird,
 b) das Produkt $i_n = m_n \cdot p_n$ aus dem über die Kapillarzonenelektrophorese erhaltenen prozentualen Peakflächen-Anteil p der Isoform n ($p_n$) mit einem Multiplikator $m_n$ gebildet wird, wobei n eine ganze Zahl von 1 bis 14, $m_n = x_n \cdot n$ und $x_n$ eine beliebige Zahl aus der Gruppe der reellen Zahlen außer 0 ist,
 c) die so erhaltenen Produkte $i_1$ bis $i_n$ zur Isoformenzahl I

$$I = \sum_{i=1}^{i=n} i_n$$

aufsummiert werden, und
 d) die erhaltene Isoformenzahl I des besagten Sialoglycoproteins mit der Isoformenzahl I eines Sialoglycoprotein-Standards verglichen wird.

2. Verfahren nach Anspruch 1, **dadurch gekennzeichnet, dass** das Sialoglycoprotein ein humanes Sialoglycoprotein ist.

3. Verfahren nach einem der vorhergehenden Ansprüche, **dadurch gekennzeichnet, dass** das Sialoglycoprotein ein rekombinantes humanes Sialoglycoprotein ist.

**4.** Verfahren nach einem der vorhergehenden Ansprüche, **dadurch gekennzeichnet, dass** das Sialoglycoprotein Erythropoietin ist.

**5.** Verfahren nach einem der Ansprüche 1 bis 3, **dadurch gekennzeichnet, dass** das Sialoglycoprotein Follikel-stimulierendes Hormon ist.

**6.** Verfahren nach einem der Ansprüche 1 bis 3, **dadurch gekennzeichnet, dass** das Sialoglycoprotein Chorion-Gonadotropin ist.

**7.** Verfahren nach einem der vorhergehenden Ansprüche, **dadurch gekennzeichnet, dass** n eine ganze Zahl von 1 bis 8 ist.

**8.** Verfahren nach einem der vorhergehenden Ansprüche, **dadurch gekennzeichnet, dass** $x_n = 1$ ist.

**9.** Verfahren nach einem der vorhergehenden Ansprüche, **dadurch gekennzeichnet, dass** sich $x_n$ aus der spezifischen Aktivität der jeweiligen Isoform n errechnet bzw. dass in $x_n$ die spezifische Aktivität der jeweiligen Isoform n eingeht; in diesem Falle ist n bevorzugt n = 1.

**10.** Verfahren nach einem der vorhergehenden Ansprüche, **dadurch gekennzeichnet, dass** die Isoformenzahl I ein Qualitätsmerkmal für die biologische Wirksamkeit des Sialoglycoproteins darstellt.

**11.** Verfahren nach dem vorhergehenden Anspruch, **dadurch gekennzeichnet, dass** man die biologische Wirksamkeit des Sialoglycoproteins durch Vergleich mit der bestimmten Isoformenzahl eines Sialoglycoprotein-Standards bewertet.

**12.** Verfahren nach einem der vorhergehenden Ansprüche, **dadurch gekennzeichnet, dass** die Isoformenzahl I ein Qualitätsmerkmal für die Chargenkonsistenz (Batch-to-Batch Consistency) des Sialoglycoproteins darstellt.

**13.** Verfahren nach dem vorhergehenden Anspruch, **dadurch gekennzeichnet, dass** man die Chargenkonsistenz des Sialoglycoproteins durch Vergleich mit der bestimmten Isoformenzahl eines Sialoglycoprotein-Standards bewertet.

**14.** Verfahren nach einem der vorhergehenden Ansprüche, **dadurch gekennzeichnet, dass** die Isoformenzahl I ein Qualitätsmerkmal für die Lagerstabilität des Sialoglycoproteins darstellt.

**15.** Verfahren nach dem vorhergehenden Anspruch, **dadurch gekennzeichnet, dass** man die Lagerstabilität des Sialoglycoproteins durch Vergleich mit der bestimmten Isoformenzahl eines Sialoglycoprotein-Standards bewertet.

**16.** Verfahren nach einem der vorhergehenden Ansprüche, **dadurch gekennzeichnet, dass** die Isoformenzahl I ein Qualitätsmerkmal für die biologische Halbwertszeit des Sialoglycoproteins darstellt.

**17.** Verfahren nach dem vorhergehenden Anspruch, **dadurch gekennzeichnet, dass** man die biologische Halbwertszeit des Sialoglycoproteins durch Vergleich mit der bestimmten Isoformenzahl eines Sialoglycoprotein-Standards bewertet.

**18.** Verwendung der über das Verfahrens nach einem der Ansprüche 1 bis 17 ermittelten Isoformenzahl I als Qualitätsmerkmal für die biologische Wirksamkeit des Sialoglycoproteins, als Qualitätsmerkmal für die Chargenkonsistenz (Batch-to-Batch Consistency) des Sialoglycoproteins, als Qualitätsmerkmal für die Lagerstabilität des Sialoglycoproteins oder als Qualitätsmerkmal für die biologische Halbwertszeit des Sialoglycoproteins.

**Claims**

**1.** Method for characterisation of the quality of sialoglycoproteins by an isoform number I, in which

a) the sialoglycoprotein is separated by means of capillary zone electrophoresis,
b) the product $in = m_n \cdot p_n$ is formed from the percentage peak area component p of the isoform n ($p_n$), which is obtained via capillary zone electrophoresis, with a multiplier $m_n$, n being a whole number from 1 to 14, $m_n = x_n \cdot n$ and $x_n$ any number from the group of real numbers apart from 0, and

c) the thus obtained products $i_1$ to in are summated to form the isoform number I

$$I = \sum_{i=1}^{i=n} i_n$$

d) the obtained Isoform number I of said sialoglycoprotein is compared with the Isoform number I of a sialoglycoprotein standard.

2. Method according to claim 1, **characterised in that** the sialoglycoprotein is a human sialoglycoprotein.

3. Method according to one of the preceding claims, **characterised in that** the sialoglycoprotein is a recombinant human sialoglycoprotein.

4. Method according to one of the preceding claims, **characterised in that** the sialoglycoprotein is erythropoietin.

5. Method according to one of the preceding claims, **characterised in that** the sialoglycoprotein is follicle-stimulating hormone.

6. Method according to one of the preceding claims, **characterised in that** the sialoglycoprotein is chorionic gonadotropin.

7. Method according to one of the preceding claims, **characterised in that** n is a whole number from 1 to 8.

8. Method according to one of the preceding claims, **characterised in that** $x_n = 1$.

9. Method according to one of the preceding claims, **characterised in that** $x_n$ is calculated from the specific activity of the respective isoform n or **in that** the specific activity of the respective isoform n goes into $x_n$.

10. Method according to one of the preceding claims, **characterised in that** the isoform number I represents a quality feature for the biological effectiveness of the sialoglycoprotein.

11. Method according to the preceding claim, **characterised in that** the biological effectiveness of the sialoglycoprotein is assessed by comparison with the specific isoform number of a sialoglycoprotein standard.

12. Method according to one of the preceding claims, **characterised in that** the isoform number I represents a quality feature for the batch-to-batch consistency of the sialoglycoprotein.

13. Method according to the preceding claim, **characterised in that** the batch consistency of the sialoglycoprotein is assessed by comparison with the specific isoform number of a sialoglycoprotein standard.

14. Method according to one of the preceding claims, **characterised in that** the isoform number I represents a quality feature for the storage stability of the sialoglycoprotein.

15. Method according to the preceding claim, **characterised in that** the storage stability of the sialoglycoprotein is assessed by comparison with the specific isoform number of a sialoglycoprotein standard.

16. Method according to one of the preceding claims, **characterised in that** the isoform number I represents a quality feature for the biological half-life of the sialoglycoprotein.

17. Method according to the preceding claim, **characterised in that** the biological half-life of the sialoglycoprotein is assessed by comparison with the specific isoform number of a sialoglycoprotein standard.

18. Use of the method according to one of the claims 1 to 17 as quality feature for the biological effectiveness of the sialoglycoprotein, as quality feature for the batch-to-batch consistency of the sialoglycoprotein, as quality feature for the storage stability for the sialoglycoprotein, or as quality feature for the biological half-life of the sialoglycoprotein.

**Revendications**

1. Procédé pour caractériser la qualité de sialoglycoprotéines par le biais d'un nombre d'isoformes I, dans lequel :

   a) la sialoglycoprotéine est séparée par électrophorèse capillaire de zone,
   b) le produit $i_n = m_n \cdot p_n$ est obtenu en multipliant la fraction p, exprimée en pourcentage, de la surface de pic de l'isoforme n (pn) obtenu par électrophorèse capillaire de zone, par un facteur $m_n$, dans lequel n représente un nombre entier de 1 à 14, $m_n = x_n \cdot n$ et $x_n$ représente n'importe quel nombre de l'ensemble des nombres réels, excepté 0,
   c) les produits $i_1$ à $i_n$ obtenus de la sorte sont additionnés de manière à fournir le nombre d'isoformes I,

   $$I = \sum_{i=1}^{i=n} i_n$$

   et
   d) le nombre d'isoformes I obtenu de ladite sialoglycoprotéine est comparé au nombre d'isoformes I d'une sialoglycoprotéine de référence.

2. Procédé selon la revendication 1, **caractérisé en ce que** la sialoglycoprotéine est une sialoglycoprotéine humaine.

3. Procédé selon l'une quelconque des revendications précédentes, **caractérisé en ce que** la sialoglycoprotéine est une sialoglycoprotéine humaine recombinante.

4. Procédé selon l'une quelconque des revendications précédentes, **caractérisé en ce que** la sialoglycoprotéine est l'érythropoïétine.

5. Procédé selon l'une quelconque des revendications 1 à 3, **caractérisé en ce que** la sialoglycoprotéine est l'hormone de stimulation des follicules.

6. Procédé selon l'une quelconque des revendications 1 à 3, **caractérisé en ce que** la sialoglycoprotéine est la gonadotropine chorionique.

7. Procédé selon l'une quelconque des revendications précédentes, **caractérisé en ce que** n est un nombre entier de 1 à 8.

8. Procédé selon l'une quelconque des revendications précédentes, **caractérisé en ce que** $x_n = 1$.

9. Procédé selon l'une quelconque des revendications précédentes, **caractérisé en ce que** l'on calcule $x_n$ à partir de l'activité spécifique de chaque isoforme n ou **en ce que** l'activité spécifique de chaque isoforme intervient dans le terme $x_n$ ; dans ce cas, la valeur de n est, de préférence, n = 1.

10. Procédé selon l'une quelconque des revendications précédentes, **caractérisé en ce que** le nombre d'isoformes I représente une marque de qualité pour déterminer l'efficacité biologique de la sialoglycoprotéine.

11. Procédé selon la revendication précédente, **caractérisé en ce que** l'on évalue l'efficacité biologique de la sialoglycoprotéine par comparaison avec le nombre d'isoformes déterminé d' une sialoglycoprotéine de référence.

12. Procédé selon l'une quelconque des revendications précédentes, **caractérisé en ce que** le nombre d'isoformes I constitue une marque qualité pour l'homogénéité des lots de sialoglycoprotéine (homogénéité d'un lot à un autre).

13. Procédé selon la revendication précédente, **caractérisé en ce que** l'on évalue l'homogénéité des lots de sialoglycoprotéine par comparaison avec le nombre d'isoformes déterminé d'une sialoglycoprotéine de référence.

**14.** Procédé selon l'une quelconque des revendications précédentes, **caractérisé en ce que** le nombre d'isoformes I représente une marque de qualité pour déterminer la stabilité au stockage de la sialoglycoprotéine.

**15.** Procédé selon la revendication précédente, **caractérisé en ce que** l'on évalue la stabilité au stockage de la sialoglycoprotéine par comparaison avec le nombre d'isoformes déterminé d'une sialoglycoprotéine de référence.

**16.** Procédé selon l'une quelconque des revendications précédentes, **caractérisé en ce que** le nombre d'isoformes I représente une marque de qualité pour la demi-vie biologique de la sialoglycoprotéine.

**17.** Procédé selon la revendication précédente, **caractérisé en ce que** l'on évalue la demi-vie biologique de la sialoglycoprotéine par comparaison avec le nombre d'isoformes déterminé d'une sialoglycoprotéine de référence.

**18.** Utilisation du nombre d'isoformes I établi au moyen du procédé selon l'une quelconque des revendications 1 à 17, comme marque de qualité pour l'efficacité biologique de la sialoglycoprotéine, comme marque de qualité pour l'homogénéité de lots de sialoglycoprotéine (homogénéité d'un lot à l'autre), comme marque de qualité pour la stabilité au stockage de la sialoglycoprotéine ou comme marque de qualité pour la demi-vie biologique de la sialoglycoprotéine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0428267 B1
- US 421444 A
- EP 0843821 B1
- DE 19527054
- EP 1996 B1
- US 1989 A

**In der Beschreibung aufgeführte Nicht-Patentliteratur**

- **M-E. Behr-Gross ; A. Daas ; A.F. Bristow.** Collaborative study for the establishment of erythropoietin BRP batch 2. *Pharmeuropa Bio,* Januar 2004, 23-33
- **Hermentin et al.** *Glycobiology,* vol. 6, 217-230